# EUROPEAN PATENT APPLICATION

(11) **EP 3 335 794 A1**
(43) Date of publication of application: **20.06.2018**
(21) Application number: 17207144.1
(22) Date of filing: 13.12.2017
(51) Int. Cl.: B01L 3/00, G01N 33/49

(54) **DEVICE AND METHOD FOR DETECTING THE DEPOSITION OF A BIOLOGICAL LIQUID SAMPLE ON A SUBSTRATE**

(30) Priority: 13.12.2016 WO PCT/IB2016/057561
(71) Applicant: DBS System SA, 1196 Gland (CH)
(72) Inventor: BÉGUIN, Steve, Springvale, Victoria 3171 (AU); DÉGLON, Julien, 1007 Lausanne (CH); THOMAS, Aurélien, 1196 Gland (CH)
(74) Representative: Grosfillier, Philippe

(57) **Abstract**

A device for detecting the deposition of a biological liquid sample for subsequent analysis, the device including a substrate for receiving the biological liquid sample, and two electrodes, each electrode having a free end, the respective free ends configured to be near or in contact with a surface of the substrate, the distance between the electrodes being such that the sample or at least a part of the sample is located between them.

## Description

### FIELD OF THE INVENTION

The present invention relates to the manipulation of biological liquid samples, such as blood, for subsequent analysis. It more precisely relates to the detection of a deposition of a biological liquid sample on a substrate.

### BACKGROUND

Current technologies in microfluidic systems focus mainly on molecule, particle or cell detection. The methods used for those applications typically involve the quantification of physical parameters such as a molecule concentration or the presence of specific cell type. In larger systems, detection of presence or quantity of liquid in a reservoir, a pipe, or a channel has been addressed using a wide variety of approaches and sensors. Common techniques involve optical transducers, electrochemical sensing systems, capacitively or inductively coupled detectors, conductivity sensors, among others. But the need to detect the act of transferring a small liquid sample on a receiving porous substrate is confined to applications where a sample has to be collected for further analysis, and has not yet been described. Nowadays trend in lab on chip systems, sample preparation devices, and point of care devices is to perform multiple steps sequentially. Those steps typically include sample insertion in the device, metering, enrichment or depletion of sample constituents, filtration or separation of solid and liquid fraction, analysis, extraction or ejection of processed sample, etc. The detection of those steps plays an important role to ensure the standardization of samples and sampling procedure by enabling the minimization of pre-analytical variability, which is of paramount importance for applications where a quantitative measurement is to be performed on the sample considered. See for example Giuseppe Lippi et al., "Pre-analytical variability and quality of diagnostic testing. Looking at the moon and gazing beyond the finger," New Zealand Journal of Medical Laboratory Science, Vol. 69, No. 1, April 2015, pp. 4-8, this reference herewith incorporated by reference in its entirety.

Sample preparation at point of collection (SPPOC), a subset of Point Of Care (POC) devices, is attracting significant attention due to its cost effective pre-processing of samples to be analyzed. Typical advantage of sample preparation is the stabilization of sample to be analyzed for storage at room temperature, reduction of material to be transported, inactivation of biological hazards, purification of the sample, enrichment of molecules of interest, etc. A concrete example of SPPOC is the dry blood spot (DBS) and dry plasma spot (DPS) technologies, where a dry biological spot of fluid is formed on a substrate at the point of collection, for further analysis in a laboratory.

The DBS sampling process is less invasive than conventional blood sampling because only a small volume (i.e. 20µL) is collected and spotted onto a filter paper card after a small fingerprick. Because of the ease of collection, DBS can be obtained in a non-hospital environment by minimally trained technicians or potentially even at home by the patients themselves. In addition, most of the pathogenic agents are deactivated on the filter paper during drying, reducing the risk of infection to a minimum, whereas the analytes of interest are stable over further months at room temperature. Because blood can be maintained on a credit-card format sample at room temperature, the cost of shipping and storage of filter paper cards is substantially reduced.

The first biomedical application of DBS on filter paper dates to 1963 when Professor Robert Guthrie introduced this alternative sampling method for detection of phenylketonuria in the newborn population. Detection of L-phenylalanine was based on a microbiological test that was sufficiently sensitive but with low analytical throughput. In the early 1990s, the development of PCR and immunoassays, including ELISA, RIA, or FIA, enabled the detection of DNA, viral RNA, antibodies, and hormones from DBS with an acceptable waiting time that was suitable for high-throughput analyses. More recently, DBS sampling form has been successfully applied to the monitoring of therapeutic agents, pharmacokinetic, and toxicokinetic studies based on liquid chromatography mass spectrometry (LC-MS). These combined advantages make the DBS procedure a patient-friendly tool for blood collection, especially in problematic and vulnerable patient populations. The ease of the process can also help in the recruitment of subjects (human and animal) for preclinical and clinical studies. Despite the above-discussed advantages, DBS sampling is rarely implemented in drug development, clinical analysis nor in a broader extent to people care. Filter paper is indeed a passive support that requires external manipulations to obtain accurate volume measurement and plasma analysis. Taken together, these external steps are tedious and make the DBS not competitive enough compared to conventional venipuncture.

International patent publication No. WO2013/144743 describes a lab-on-chip-based system for the direct and multiple sampling, control of the volume, fluid filtration, biochemical reactions, sample transfer, and dried spot generation on the conventional and commercial cards for dried fluid spot. Within an all-in-one integrated holder, this disclosure allows the complete process required to ensure a quantitative analysis of blood, plasma or any other fluids, modification and enrichment of molecule subsets, and formation of a dried fluid spot on the specific spot location of a passive cellulose, non-cellulose, absorbent, or non-absorbent membrane material sampling. International patent publication No. WO2015/140740 further describes a passive method to separate plasma or serum from whole blood and the active ejection of a metered pre-processed sample on a receiving substrate.

Whereas the above cited patent documents and related products solve the issue of quality and accuracy of the sample to be analyzed, the precise detection of the time the sample has been collected as well as the storage conditions are still limiting factors. Indeed, there is a strong need to reliably record the time of sample collection for applications such as but not limited to pharmacokinetics, optimization of drug posology, clinical trials. For those applications, the patient may be required to provide biological fluid samples multiple times per day or week.

### SUMMARY OF INVENTION

According to one aspect of the present invention, a device for detecting the deposition of a biological liquid sample for subsequent analysis is provided. Preferably, the device including a substrate for receiving the biological liquid sample, and two electrodes, each electrode having a free end, the respective free ends configured to be near or in contact with a surface of the substrate, the distance between the electrodes being such that the sample or at least a part of the sample is located between them.
According to another aspect of the present invention, a method for detecting and recording the deposition of a biological liquid sample on a substrate for subsequent analysis is provided. Preferably, the method includes the steps of (a) transferring the biological liquid sample onto a substrate, (b) locating at least a part of the sample between electrodes, and (c) performing at least one of conductive and capacitive measurement with the electrodes to detect a presence of the biological liquid sample on the substrate.

The above and other objects, features and advantages of the present invention and the manner of realizing them will become more apparent, and the invention itself will best be understood from a study of the following description with reference to the attached drawings showing some preferred embodiments of the invention.

### BRIEF DESCRIPTION OF THE SEVERAL VIEWS OF THE DRAWINGS

The above and other objects, features and advantages of the present invention and the manner of realizing them will become more apparent, and the invention itself will best be understood from a study of the following description with reference to the attached drawings showing some preferred embodiments of the invention.

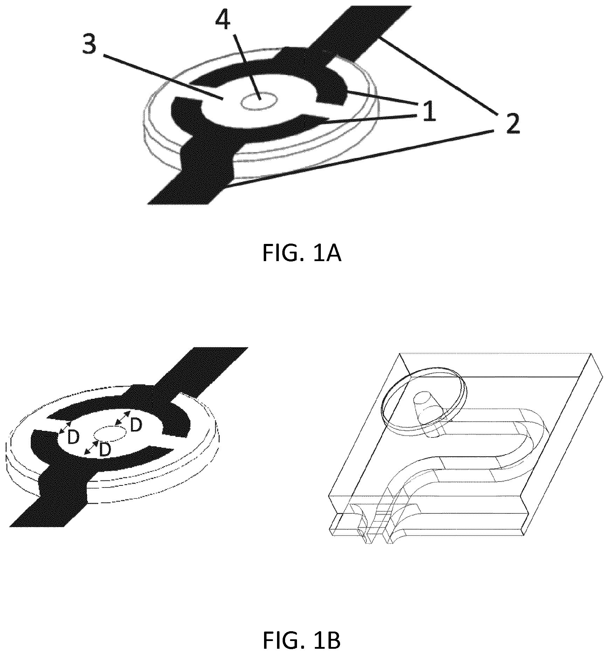

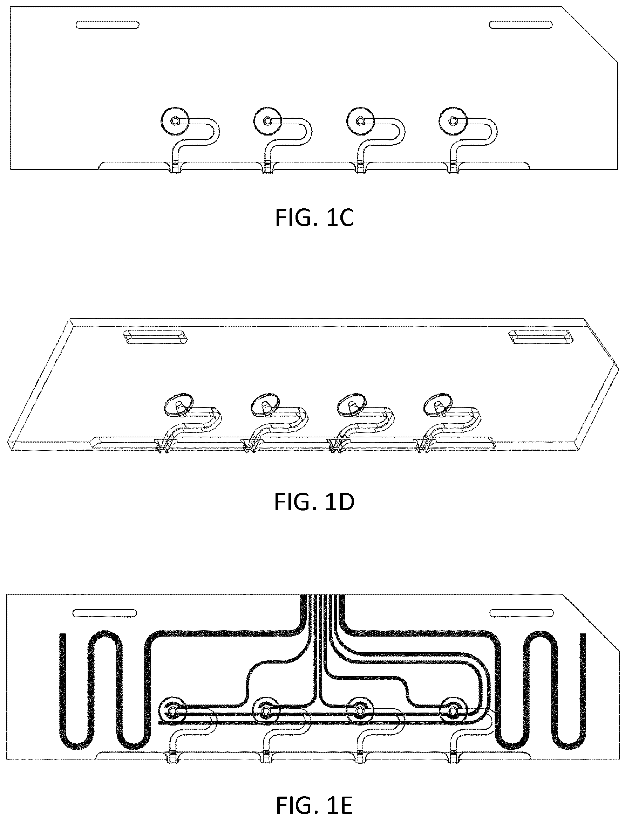

FIGs. 1A to IE show different views of the electrodes 1 that are configured to detect presence of a spot on a solid substrate 3 as an exemplary representation, with FIG. 1A showing a pair of electrodes 1 and conductive tracks 2 on a solid substrate 3, with hole or opening 4 in the solid substrate 3 to feed fluid for transfer, FIG. 1B showing distance D between the electrodes 2 and the opening 4, and an arrangement of substrate 3 and electrodes 2 on top of a device having a fluidic channel in a body for delivering a sample, FIGs. 1C and 1D show different views of a plurality of fluidic channels in a body of a sampling device 12, in the variant shown four (4) fluidic channels in a body of a sampling device 12 each associated with electrodes and a solid substrate 3, and FIG. IE shows an exemplary view of the sampling device 12 with the electrode tracks 2, electrodes 1, excitation electrode 9, and antenna 15;

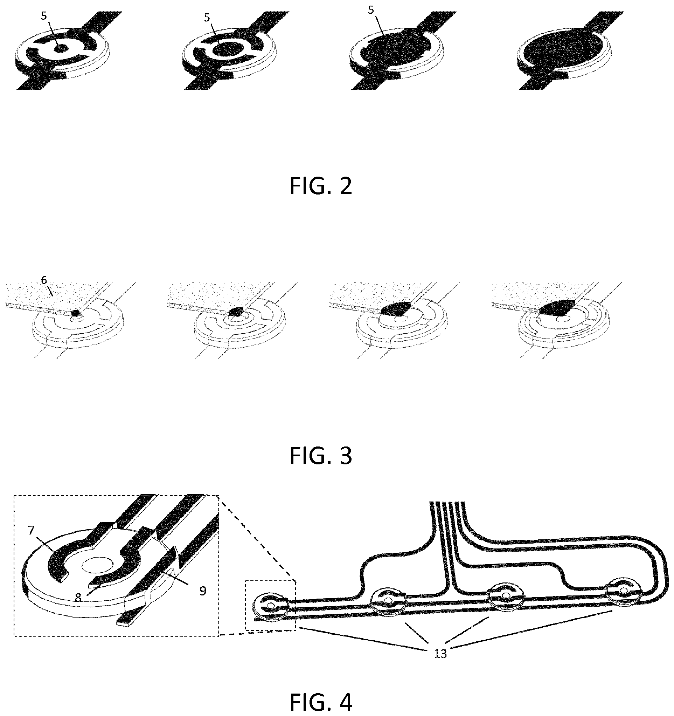

FIG. 2 is an exemplary representation of different stages of the pair of electrodes 1 as a sequence representing the transfer and spread of a conductive liquid 5, for example the biological liquid sample, on a solid insulator substrate, lowering electric resistance and granting electric contact between the electrodes;
FIG. 3 is an exemplary representation of different stages of the pair of electrodes 1 as a sequence representing the transfer of conductive liquid in a porous insulator substrate 6, lowering electric resistance of the porous substrate and granting electric contact between the electrodes. In this example, for illustration purposes, only a quarter of the porous insulator substrate 6 is displayed to illustrate the spread of the liquid in one quadrant of the porous media;
FIG. 4 represents an embodiment with four sensing units 13 and including three electrodes per sensing unit. In this configuration a sensing electrode 7 is completed by a reference electrode 8 to enable the use of a compensation circuitry, and an excitation electrode 9 to operate the measurements;

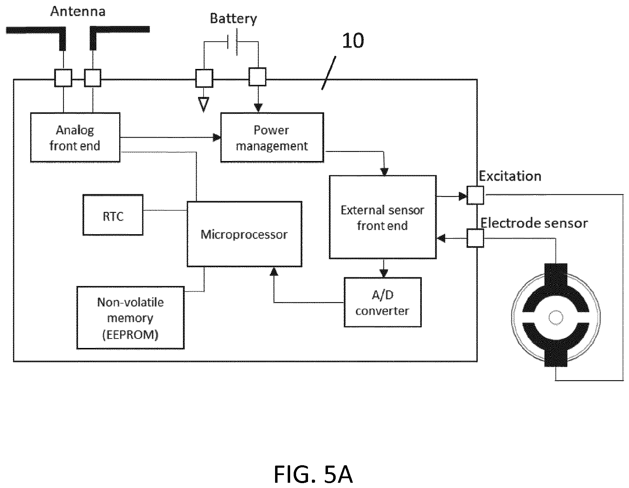

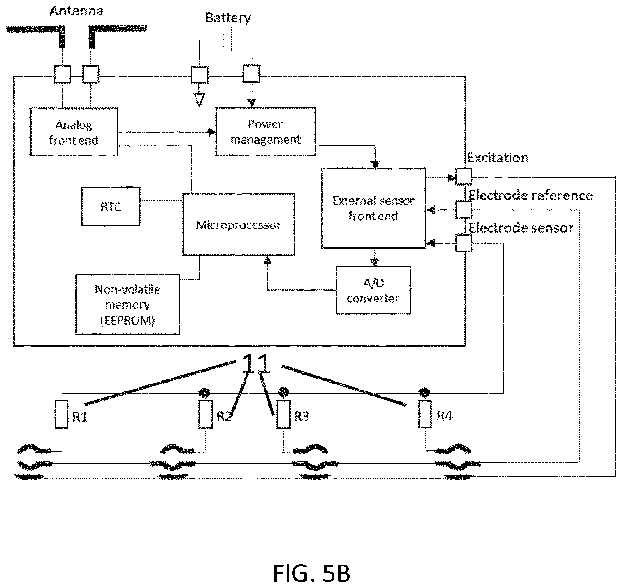

FIGs. 5A and 5B show different configurations of data logging and processing systems that are in operative connection with the pair of electrodes 1, with FIG. 5A showing a configuration with single electrode pair connected to a radio-frequency identification (RFID) data logging system on a chip 10, and FIG. 5B showing a configuration with four (4) sensing units composed of three (3) electrodes, with the sensing electrode connected to an identification circuitry 11 formed by four different resistances, allowing to backtrack the sensing unit that triggered a specific logging event;

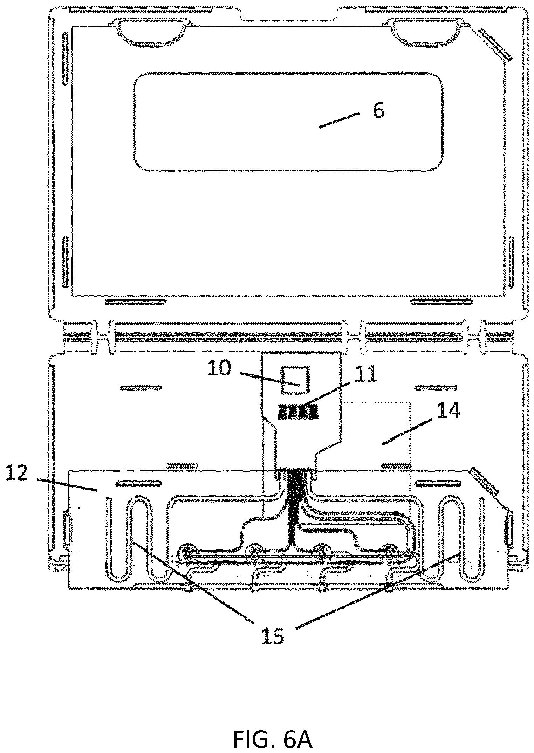

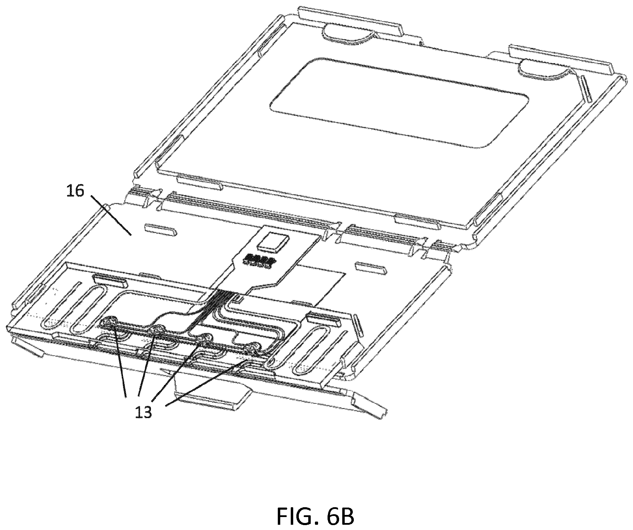

FIGs. 6A and 6B represent an embodiment where sensing units are placed at the outlets of a sampling device 12 including four (4) microfluidic channels for sample collection and transfer on receiving substrate 6, held together by a casing 16. The sensing electrodes are connected to the identification circuitry 11. Sensing units 13, identification circuitry 11, and antenna 15 are connected to a RFID datalogging system on a chip 10 powered by a battery 14; and

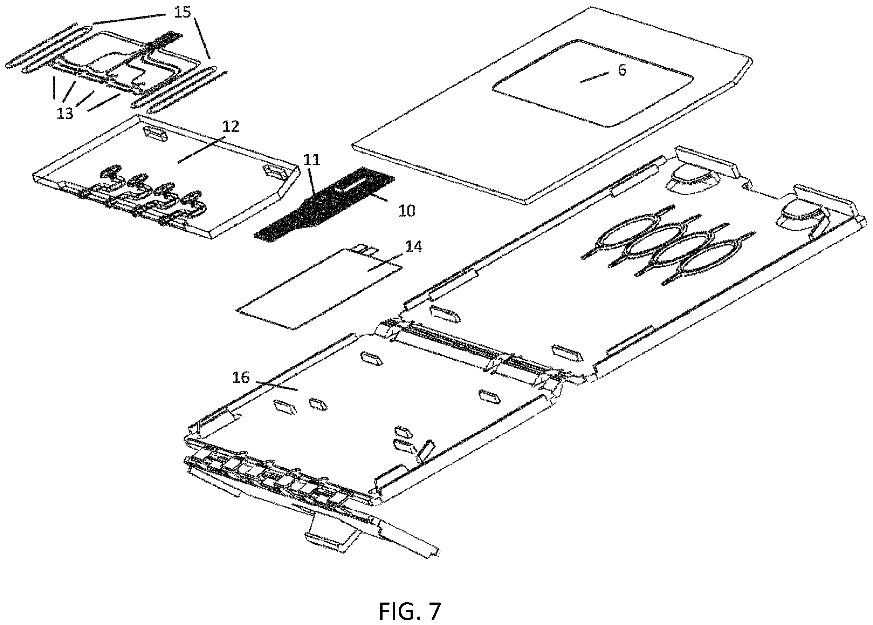

FIG. 7 is an exploded view of the embodiment described in FIGs. 6A and 6B.

Herein, identical reference numerals are used, where possible, to designate identical elements that are common to the figures. Also, the images are simplified for illustration purposes and may not be depicted to scale.

### DESCRIPTION OF THE PREFERRED EMBODIMENTS

According to one aspect of the present invention, the herein presented device for detecting the deposition of a biological liquid sample, and method of operating the same, provides several improvements with respect to the state of the art. To this effect, according to an aspect, the invention relates to a device for detecting the deposition of a biological liquid sample on a substrate for subsequent analysis, the device comprising two electrodes with respective free ends that are designed to be near or in contact with the surface of the substrate, the distance between the electrodes being such that the sample or at least a part of it may be located between them, as shown in FIGs. 1A-1E and 3. Configuration of outlet 4, electrodes 1, and receiving substrate 3 are sized and positioned as to have the fluid significantly modifying the impedance of the space between the electrodes 1, occupied either by air or by the receiving substrate, during or after the fluid has been transferred to the receiving substrate. Indicative preferred dimensions of the elements are given, but not limited to:
Width of outlet 4 is preferably between 0.1 mm to 2 mm;
Spacing between electrodes 1 on solid substrate 3 is preferably between 0.2 mm to 5 mm, indicated as distance D;
Spacing between electrodes 1 and outlet 4 is preferably between 0.2 mm - 5 mm, indicated as distance D;
Thickness of electrodes 1 and conductive tracks 2 is preferably between 0.1 µm - 200 µm;
Gap between the receiving substrate 3 and the outlet 4 to initiate the transfer of the fluid is preferably below 200 µm;

According to another aspect of the present invention, a home-based self-use blood collection device is presented, that has simplified logistics and cost effectiveness. Then, the device and method allows to relieve the burden of correct labeling, e.g. date, time, study number, from the patient, the device and method provides for a new tool to standardize and authenticate the collected samples, preventing wrong results caused by human. Also, the invention relates to a method of operating the device, and a system including at least one device as described above. In particular, according to another aspect of the present invention, the device and the method preferably focus on the direct detection of the act of transferring a pre-processed sample on a receiving substrate. The advantage of transferring the sample on a substrate being its long-term storage ability as a dried bio-fluid spot.

In a preferred embodiment the device furthermore includes a data logger unit that is adapted to at least record the time when the sample 5 has been positioned between the electrodes 1. A change in impedance between the electrodes 1, beyond a predefined threshold, can be detected by the data logger unit, for example but not limited to a microprocessor having memory, communication interface, and a Real-Time Clock (RTC) associated thereto, and the data logger unit can be configured to trigger an interrupt. Upon detection of the interrupt, the data logger unit can be configured to measure the value of the impedance at electrodes 1 along with timestamp of from the embedded RTC and record them in non-volatile memory of the data logger unit. Such device is schematically shown in FIGs. 5A and 5B. Other parameters may also be recorded in the data logger, such as temperature, relative humidity or room pressure. Different types of electrical measurement can be made with the electrodes but preferably conductive or capacitive measurement is chosen. The substrate is preferably an absorbing material but any other suitable material can be used. With an absorbing material, a dried spot may be generated, which facilitates the subsequent analysis. Also, any biological fluid can be used, for example but not limited to blood, plasma, serum, urine, tear, sputum, if a conductive path can be established between the electrodes.

The conductance-based detection of the act of transferring the fluid sample on the substrate can be used to trigger various actions such as the recording of a time stamp in the data logger to capture the time the sample has been spotted, to trigger the beginning of recording of storage parameters, for example but not limited to temperature, relative humidity, or to give a signal to inform the user that the transfer has successfully occurred or for the integration in an automated quality control system in a production line. By using different set of resistors connecting the sensing electrodes and the conductance meter of a data logger, it is possible to spatially discriminate where the transfer occurred. In the context of a device containing multiple fluidic channels, as shown in FIG. 6A and 6B, this setup allows to generate a time stamp when considered fluid is transferred on desired substrate with related electrical resistance/conductance/impedance identifying the set of channels that have contributed to the transfer.

Also, according to another aspect of the present invention, a method for detecting and recording the deposition of a biological liquid sample on a substrate for subsequent analysis is provided, the method comprising the use of the device as described herein in the various embodiments. Preferably, the method includes the following steps:
(a) transferring the biological liquid sample onto the substrate 3, for example via fluidic channels of the sampling device 12:
(b) locating at least a part of the sample 5 between the electrodes 1; and
(c) performing a conductive or capacitive measurement with the electrodes 1, to detect presence of the sample 5.

In a preferred embodiment, the method furthermore comprises a step of recording of at least the time when the sample 5 has been positioned between the electrodes 1.

According to one aspect of the present invention, the method allows a precise detection of the time a sample is transferred on a desired substrate. The sensor can be highly integrated and miniaturized, and can be integrated either on the receiving substrate or on the dispensing element of a SPPOC, POC, In Vitro Diagnostics (IVD), or lab-on-chip device. The sensor provides a reliable readout that transfer from collection or sample pre-processing region of a device onto a receiving substrate has occurred, without the need for calibration or fine tuning of the sensing elements.

According to yet another aspect of the invention, the device may be advantageously used for the detection of transfer of fluid samples containing biological material, for example but not limited to cells, proteins, molecules, etc. from metering fluidic channel onto a filter paper and consequent generation of dry spots.

In another embodiment, the device and method may be used for the detection of transfer of biological fluid samples, for example but not limited to blood, plasma, serum, urine, saliva, sputum, tear, semen, interstitial fluid, from metering fluidic channel onto a filter paper and consequent generation of dry spots.

Another preferred application of the device is the detection of transfer of blood, plasma and serum samples from metering microfluidic channels onto a filter paper and consequent generation of dry blood spots.

The device allows the quantitative analysis of molecules and molecule kinetics on conventional dried-spots sampling cards. Receiving substrate is typically a flat and open surface, but can also be contained within a channel, reservoir, or holding element. Substrate material can be cellulose, non-cellulose, absorbent, or non-absorbent material.

As an example, the device can be designed to use the most conventional dried spot sampling substrates including #903™ brand paper (Whatman, Inc., New Jersey USA), bond elut dried matrix spotting (Agilent, Germany) or treated filter papers, such as FTA and FTA Elute brand paper or DMPK A, B or C card (Whatman, Inc., New Jersey USA).

According to yet another aspect of the invention, the device includes a sensor having a sensing element including at least one of the following features:
At least two conducting or capacitively coupled electrodes 1 placed near of the outlet 4 of the dispensing element, the distance D between outlet 4 and electrodes 1 being preferably smaller than 1 mm, for example as shown in FIG. 1B. The distance D is defined as the distance that the biological liquid sample will travel in the substrate 6, after having been received by a dispensing element, to establish electrical conductivity between electrodes 1, such that a detection of the biological liquid sample 5 5 can be detected, to generate a trigger in the data logger unit.

FIGs. 2-3 shows different stages of the pair of electrodes 1 as a sequence representing the transfer and spread of biological liquid sample 5 inside the substrate 6. First, biological liquid sample 5 has been deposited or otherwise put into contact with the substrate 6, for example by a dispensing device, such as a pipetting device, shown on the left of FIGs. 2-3. Next, due to the characteristics of the substrate 6, the biological liquid sample 5 begins to spread by capillary force provided by substrate 6, to increasingly cover an area between the two electrodes 1. In a variant, substrate 6 is not porous, and biological liquid sample 5 spreads on a surface of substrate 6 due to the hydrophilic properties of the surface. Due to the electric conductivity of biological liquid sample 5, the spreading of liquid 5 in substrate increasingly reduces an electric resistance between electrodes 1, and will provide for an electric contact between the electrodes 1. The distance D is defined as a distance biological liquid sample 5 has to travel inside or on the substrate 6 to grant electrical conductivity between the electrodes 1 and such that a detection of the biological liquid sample 5 can be made, for example by measuring a resistance between electrodes 1. In a variant, as the spreading of biological liquid sample 5 progressively changes a capacity between electrodes 1, a capacity between the electrodes 1 can be measured to detect presence of biological liquid sample 5. Preferably, distance D is chosen to be as small as possible but for preserving a gap between the electrodes 1 and the outlet 2. As shown in FIGs. IE and 4, the operation of the three (3) electrodes for detecting presence of the biological liquid sample 5 is such that there is one common electrode and two sensing electrodes (one per measurement circuit) therefore one electrode can act as a reference and the other is connected via the resistor to allow for backtrack identification.

A separation area defined by distance D around the outlet that is not covered by a conductive material in physical or electrical contact of the sample to be transferred ensures an event is detected only when transfer of the sample to the substrate 3 has occurred. In a variant, electrodes 1 do not necessarily have to be separated by the outlet 4 in between, and the electrodes 1 could be placed on one side of outlet 4, providing equivalent working principle. At least two conducting or capacitively coupled electrodes placed near the outlet of the dispensing element, outside the channel containing the sample to be transferred, and without physical or electrical contact with the sample before the sample is transferred on receiving substrate;
A region where the substrate receives the sample, granting physical or electrical contact between the sample and the electrodes during the transfer;
At least two conducting or capacitively coupled electrodes placed in the region where the substrate receives the sample, granting physical or electrical contact to the sample after transfer has occurred;
At least two conducting electrodes placed near the outlet of the dispensing element, outside the channel containing the sample to be transferred, and without physical or electrical contact with the sample;
Multiple parallel fluidic channels, with at least a pair of electrode placed in any configuration described above;
Multiple parallel fluidic channels, with at least a pair of electrode placed in any configuration described above and an electrode common to all the channels;
Multiple parallel fluidic channels, with at least a pair of electrode placed in any configuration described above and both electrodes of the pair common to all the channels.

In one embodiment of the invention the dispensing element comprises several fluid channels placed in parallel, each of which have described sensor and different sets of resistors attached to the electrical circuit of the electrodes to discriminate the electrode or set of electrodes contributing to the signal detected by measuring the impedance of the circuit at selected frequency.

In another embodiment, the substrate alone has low electric conductivity and the fluid has a higher electric conductivity. Thus, the transfer of the conductive fluid induces a lowering in electric resistivity or impedance of the medium that is measured between the integrated electrodes.

In another embodiment, the substrate alone has low electric resistivity and the fluid has a lower electric conductivity. Thus, the transfer of the conductive fluid induces a lowering in electric conductivity or conductance of the medium that is measured between the integrated electrodes. In another embodiment, the substrate is a porous medium with low electric conductivity and the fluid is a biological fluid with higher electric conductivity. Thus, the transfer of the conductive fluid induces a lowering in electric resistivity or impedance of the medium that is measured between the integrated electrodes.

Moreover, in another embodiment, the measurement is performed through the electrodes only when triggered by a specific event such as but not limited to:
(a) button being pressed.
(b) a contact or micro-contact being activated.
(c) a specific time or duration being reached.
(d) a combination of any of the above.

In another embodiment, when the reading from the electrodes is within predefined boundaries, the recording of a timestamp is triggered to register the date and time the sampling was performed.

In another embodiment, when the reading from the electrodes is within predefined boundaries, the recording of environmental parameters is triggered such as but not limited to:
(a) Temperature.
(b) Humidity.
(c) Electromagnetic radiation, for example but not limited to visible light, UV, IR, X rays, gamma rays.
(d) Any combination of the above.

The electric based measurement is performed through the electrodes to discriminate the presence or absence of the sample on the receiving substrate. The electric based measurement can be based on at least one of the following list:
(i) a conductive measurement where a known electrical current is being applied to and the voltage drop between the electrodes is measured.
(ii) a conductive measurement where a known electrical voltage is being fixed between the electrodes and the current flowing through the electrodes is being measured.
(iii) any of the above conductive measurement measured in DC mode.
(iv) any of the above conductive measurement measured in AC mode, at defined frequency or set of frequencies.
(v) a capacitive measurement where the capacitance between the sensing electrodes is measured.
(vi) an inductive based measurement where the inductance between the sensing electrodes is measured.
(vii) a combination of any of the above.

FIGs. 6A and 6B shows a system where different sensing units are placed at the outlets of a sampling device 12 including four (4) microfluidic channels for sample collection and transfer on receiving substrate 6, held together by a casing 16. An example of the microfluidic channels and their arrangement and principle of operation has been described in U.S. Pat. No. 9,795,960, this reference herewith incorporated by reference in its entirety. The sensing electrodes 1 that are a part of sensing units 13 are connected to the identification circuitry 11. Sensing units 13, identification circuitry 11, and antenna 15 are connected to a RFID data logging system on a chip 10 powered by a battery 14. Sampling device 12 includes microfluidic channels shown in FIGs. IB-IE, 6A-6B, and 7 each having an inlet to collect blood from a finger prick, a fluidic channel configured as a metering channel arranged between an inlet and an outlet to control the volume of the blood, and an outlet onto which a receiving substrate 3 such as a porous hydrophilic substrate can be applied to pull the metered sample out of the microfluidic channel by capillary forces.

In sum, according to some aspects of the present invention, a device and a method for detecting the transfer of a fluid sample on a substrate is provided, e.g. on an absorbing material. It may be advantageously used for the detection of the transfer of a metered biological fluid, for example but not limited to blood, plasma, serum, urine, tear, sputum, on a paper based substrate to generate dried spot for further analysis.

According to some aspects, the device and the method focus on the direct detection of the act of transferring a pre-processed sample on a receiving substrate by using an electric based measurement method using a set of electrodes. The advantage of transferring the sample on a substrate being its long-term storage ability as a dried bio-fluid spot (DBS), in the context of sample preparation at point of collection (SPPOC). The device and method advantageously allow to trace back the date and time when sampling was made as well as the recording or datalogging of environmental parameters influencing the condition of storage of the sample and which may impair the integrity of the sample prior analysis.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention not be limited to the described embodiments, and be given the broadest reasonable interpretation in accordance with the language of the appended claims

## Claims

1. A device for detecting the deposition of a biological liquid sample for subsequent analysis, the device comprising:
a substrate for receiving the biological liquid sample; and
two electrodes, each electrode having a free end, the respective free ends configured to be near or in contact with a surface of the substrate, the distance between the electrodes being such that the sample or at least a part of the sample is located between them.

2. The device of claim 1, where the substrate is porous.

3. The device according to claim 1 further comprising:
a data logger unit configured to record a time when the sample has been positioned between the electrodes.

4. The device according to claim 1, wherein the electrodes are configured to perform a conductive measurement.

5. The device according to claim 1, wherein the electrodes are configured to perform a capacitive measurement.

6. The device according to claim 1, wherein the ends of the electrodes are located on a support that includes an outlet from which the sample is configured to flow.

7. A method for detecting and recording the deposition of a biological liquid sample on a substrate for subsequent analysis, the method comprising the steps of:
(a) transferring the biological liquid sample onto a substrate;
(b) locating at least a part of the sample between electrodes; and
(c) performing at least one of conductive and capacitive measurement with the electrodes to detect a presence of the biological liquid sample on the substrate.

8. The method according to claim 6 further comprising the step of:
(d) recording a time when the sample has been positioned between the electrodes of the step (b).

9. The method according to claim 7, wherein step (a) occurs before step (b).

10. The method according to claim 7, wherein step (a) occurs after step (b).

11. The method according to claim 7, wherein the biological liquid sample includes at least one of blood, plasma, serum, urine, tear, saliva, sputum, interstitial fluid, cells, microorganisms, and bacteria.

12. The method according to claim 7, wherein the biological fluid sample is a solution including molecules of interest to be further analyzed.

13. The method according to claim 12, wherein the molecules of interest have been extracted, concentrated, and processed for further analysis.
